(19) Europäisches Patentamt
European Patent Office
Office européen des brevets

(11) **EP 4 643 880 A1**

(12) **EUROPEAN PATENT APPLICATION**
published in accordance with Art. 153(4) EPC

(43) Date of publication:
**05.11.2025 Bulletin 2025/45**

(21) Application number: **23912122.1**

(22) Date of filing: **26.12.2023**

(51) International Patent Classification (IPC):
*A61K 47/02* (2006.01)     *A23L 5/00* (2016.01)
*A61K 8/11* (2006.01)     *A61K 8/19* (2006.01)
*A61K 8/20* (2006.01)     *A61K 8/73* (2006.01)
*A61K 9/48* (2006.01)     *A61K 9/60* (2006.01)
*A61K 9/62* (2006.01)     *A61K 47/36* (2006.01)
*A61K 47/38* (2006.01)

(52) Cooperative Patent Classification (CPC):
**A23L 5/00; A61K 8/11; A61K 8/19; A61K 8/20;
A61K 8/73; A61K 9/48; A61K 9/50; A61K 47/02;
A61K 47/36; A61K 47/38**

(86) International application number:
**PCT/JP2023/046599**

(87) International publication number:
**WO 2024/143343 (04.07.2024 Gazette 2024/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **27.12.2022   JP 2022210113
23.05.2023   JP 2023084623**

(71) Applicant: **Qualicaps Co., Ltd.
Nara 639-1032 (JP)**

(72) Inventor: **HONDA, Mamoru
Yamatokoriyama-shi, Nara 639-1032 (JP)**

(74) Representative: **Kraus & Lederer PartGmbB
Thomas-Wimmer-Ring 15
80539 München (DE)**

(54) **HARD CAPSULE COMPRISING COATING CONTAINING LIGHT-BLOCKING AGENT, HARD CAPSULE PREPARATION SOLUTION, AND HARD CAPSULE PREPARATION METHOD**

(57)     An object of the present invention is to provide a hard capsule shell using calcium carbonate and having higher light-shielding ability.

A hard capsule comprising a shell containing a cellulose compound and a light-shielding agent, wherein the light-shielding agent contains calcium carbonate having a median diameter by volume of 0.7 $\mu$m to 3.8 $\mu$m, and the content of the light-shielding agent in the shell is 3 to 20 mass% when the total of shell components of the hard capsule excluding moisture is set to 100 mass%.

**(Cont. next page)**

[FIG. 1]

(A)

(B)

**Description**

Technical Field

The present description discloses a hard capsule comprising a shell containing a light-shielding agent, a hard capsule preparation solution, and a hard capsule preparation method.

Background Art

Some ingredients formulated in pharmaceuticals, quasi-drugs, foods, cosmetics, and the like are unstable to light. As a method of ensuring quality stability by preventing degradation of such ingredients due to light, a method of coating them with a shell composition having a light-shielding ability is commonly used.

Titanium oxide is generally used as the light-shielding agent. However, the titanium oxide is contraindicated for use with some ingredients formulated as pharmaceuticals because the titanium oxide impairs the stability of some of the ingredients or accelerates their degradation due to radicals generated from the titanium oxide by ultraviolet light, for example. In addition, in recent years, carcinogenicity of the titanium oxide itself has been pointed out.

In view of these circumstances, attempts have been made to formulate an ingredient other than the titanium oxide as the light-shielding agent in capsules for the pharmaceuticals, quasi-drugs, foods, cosmetics, and the like.

For example, Patent Literature 1 discloses a water-soluble metallic compound containing at least one metal selected from the group consisting of sodium, potassium, calcium, magnesium, aluminum, manganese, iron, cobalt, nickel, copper, strontium and barium, and a non-transparent shell composition containing a water-soluble cellulose derivative. Patent Literature 2 discloses a capsule forming composition containing a shell forming agent selected from the group consisting of gelatin, polysaccharide, modified starch, cellulose derivative, or synthetic polymer; and an opacifying agent in the form of calcium carbonate in an amount of between 3 and 10 wt% based on dry weight of the capsule forming formulation. In Patent Literature 2, precipitated calcium carbonate is used as the calcium carbonate. Patent Literature 3 discloses a capsule shell containing gelatin and ground calcium carbonate.

Citation List

Patent Literature

[Patent Literature 1] WO2008/156027
[Patent Literature 2] WO2019/219693
[Patent Literature 3] WO2021/069590

Summary of Invention

Technical Problem

However, as described in Patent Literature 2, the light-shielding ability of the capsule shell using the calcium carbonate as the light-shielding agent is not as high as when the titanium oxide is used.

An object of the present invention is to provide a hard capsule shell using the calcium carbonate and having higher light-shielding ability. In addition, another object is to provide the hard capsule shell using the calcium carbonate and maintaining the shell strength while maintaining the light-shielding ability.

Solution to Problem

The present invention includes the following embodiments as an example.

Item 1.

A hard capsule comprising a shell containing a cellulose compound and a light-shielding agent, wherein

the light-shielding agent contains calcium carbonate having a median diameter by volume of 0.7 $\mu$m to 3.8 $\mu$m, and
the content of the light-shielding agent in the shell is 3 to 20 mass% when the total of shell components of the hard capsule excluding moisture is set to 100 mass%.

Item 2.

The hard capsule according to item 1, wherein the cellulose compound is hydroxypropyl methylcellulose.

Item 3.

The hard capsule according to item 2, wherein the shell further contains (1) a gelling agent, or (2) the gelling agent and a gelling aid.

Item 4.

A hard capsule preparation solution containing a cellulose compound, a light-shielding agent and an aqueous solvent, wherein

the light-shielding agent contains calcium carbonate having the median diameter by volume of 0.7 $\mu$m to 3.8 $\mu$m, and the content of the light-shielding agent in the total of components of the shell excluding the aqueous solvent is 3 to 20 mass% when the total of components of the preparation solution excluding the aqueous solvent is set to 100 mass%.

Item 5.

The hard capsule preparation solution according to item 4, wherein the cellulose compound is hydroxypropyl methylcellulose.

Item 6.

The hard capsule preparation solution according to item 5, further containing (1) a gelling agent, or (2) a gelling agent and a gelling aid.

Item 7.

A preparation method for the hard capsule including the following step:
a step of preparing the hard capsule by a cooling gel method using the hard capsule preparation solution according to item 4.

Advantageous Effects of Invention

An advantage of the present invention is that it is possible to provide the hard capsule shell having higher light-shielding ability.

Brief Description of Drawings

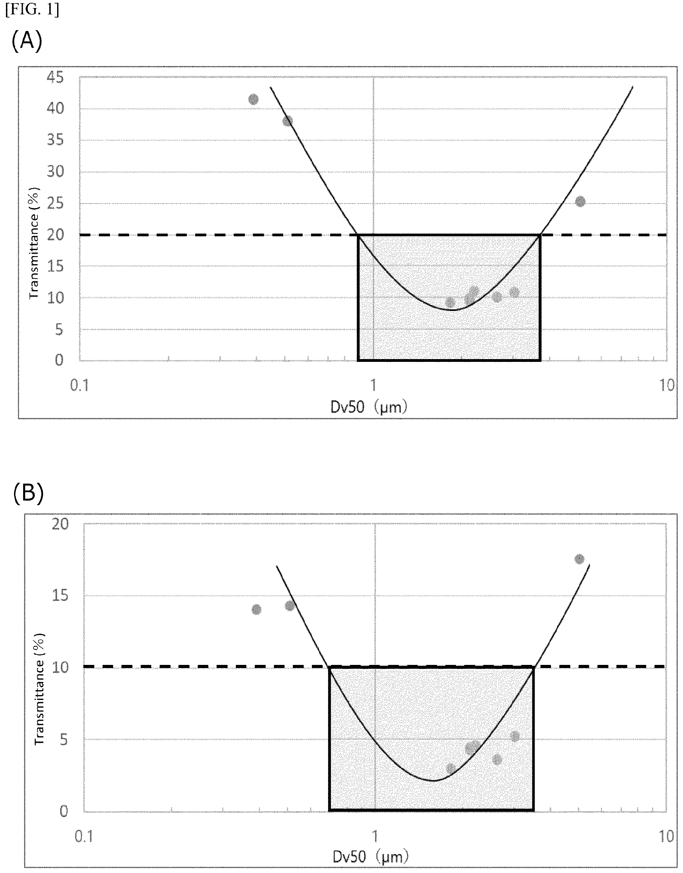

FIG. 1A is a graph showing a relationship between transmittance and Dv50 when the measurement light wavelength is 650 nm.
FIG. 1B is a graph showing a relationship between transmittance and Dv50 when the measurement light wavelength is 420 nm.

Description of Embodiments

1. Hard capsule

A hard capsule comprises a shell containing a cellulose compound and a light-shielding agent. The light-shielding agent contains calcium carbonate with a median diameter (Dv50) of 0.7 $\mu$m to 3.8 $\mu$m.
In the present description, the term "hard capsule" refers to a type of capsule in which a capsule shell is produced first and a content is filled into the produced capsule shell. Usually, the hard capsule includes a cap portion and a body portion, and is also called a hard capsule or a two-piece capsule. The "hard capsule" of the present invention does not include a soft capsule produced by filling a content between two films and bonding the films to each other, a seamless capsule produced by dropping a content together with a shell solution into a solidification liquid, and a microcapsule prepared by incorporating therein an active ingredient by precipitation or emulsification of a base material.

The cellulose compound used for the hard capsule may include a water-soluble cellulose ether in which a hydrogen atom of a hydroxy group of cellulose is substituted by at least one alkyl group or hydroxyalkyl group. Here, the "alkyl group" referred to in the above alkyl group or hydroxyalkyl group may include linear or branched lower alkyl groups having 1 to 6 carbon atoms, preferably 1 to 4 carbon atoms, specifically a methyl group, ethyl group, butyl group and propyl group. A water-soluble cellulose compound may specifically include lower alkyl celluloses such as methyl cellulose; hydroxy lower alkyl celluloses such as hydroxyethyl cellulose and hydroxypropyl cellulose; as well as hydroxy lower alkyl alkyl celluloses such as hydroxyethyl methyl cellulose, hydroxyethyl ethyl cellulose and hydroxypropyl methylcellulose (sometimes referred to herein as hypromellose or HPMC) and the like. Among these, the hydroxypropyl methylcellulose is the most suitable cellulose compound because of its excellent shell formability and excellent mechanical strength under low moisture content.

The hydroxypropyl methylcellulose used in the hard capsule includes the hypromellose specified in Table 1 as specified in the Japanese Pharmacopoeia.

Table 1

| substitution type | methoxy group | hydroxypropoxy group |
|---|---|---|
| 1828 | 16.5 - 20.0 | 23.0 - 32.0 |
| 2208 | 19.0 - 24.0 | 4.0 - 12.0 |
| 2906 | 27.0 - 30.0 | 4.0 - 7.5 |
| 2910 | 28.0 - 30.0 | 7.0 - 12.0 |

In addition, the hydroxypropyl methylcellulose includes the hypromellose having the following molecular weight, which is approved for use as a food additive in Japan.

<Molecular Weight>

Unsubstituted structural unit: 162.14
Substituted structural unit: about 180 (degree of substitution: 1.19), about 210 (degree of substitution: 2.37)
Polymer: about 13,000 (n = about 70) to about 200,000 (n = about 1000)

Commercially available hydroxypropyl methylcellulose has a ratio of weight average molecular weight (Mw) to number average molecular weight (Mn) (Mw/Mn) typically in the range of 1.5 to 4. Both the weight average molecular weight (Mw) and the number average molecular weight (Mn) for calculating the ratio (Mw/Mn) can be determined by gel chromatography (size exclusion chromatography). Principles and techniques of the gel chromatography may be referred to, but are not limited to, a description in the section "Size-Exclusion Chromatography" in the chapter "Chromatography" of the "USP30 The United States Pharmacopeia/NF25 The National Formulary".

The commercially available hydroxypropyl methylcellulose may include, TC-5 series, SB-4® series, and METOLOSE® series available from Shin-Etsu Chemical Co., Ltd., AnyCoat-C® series available from Lotte (former Samsung) Fine Chemical Co., Ltd., METHOCEL® series available from The Dow Chemical Company.

In addition, the hydroxypropyl methylcellulose used in the present invention includes a hydroxypropyl methylcellulose whose viscosity of a 2 mass% aqueous solution thereof at 20°C $\pm$ 0.1°C is in the range of 3 to 50 mPa·s.

The hydroxypropyl methylcellulose may be used as one type alone or may be used in any combination of two or more types, but in any cases, "hypromellose viscosity value" may be in the range of 300 to 5000, preferably 300 to 1500, more preferably 300 to 960. Here, "hydroxypropyl methylcellulose viscosity value" means a sum of values obtained by multiplying the viscosity (mPa·s) at 20°C $\pm$ 0.1°C of a 2 mass% aqueous solution of hydroxypropyl methylcellulose used for producing capsule film by its ratio (parts by mass) to 100 parts by mass of total amount of the hydroxypropyl methylcellulose. Specifically, when the hydroxypropyl methylcellulose whose viscosity of 2 mass% aqueous solution is 6 mPa·s is used alone to produce capsule film, the "hydroxypropyl methylcellulose viscosity value" is 600 by "6 mPa·s $\times$ 100 parts by mass". When 30 parts by mass of hydroxypropyl methylcellulose whose viscosity of 2 mass% aqueous solution is 4 mPa·s and 70 parts by mass of hypromellose whose viscosity of 2 mass% aqueous solution is 6 mPa·s are used in combination to produce capsule film, the "hydroxypropyl methylcellulose viscosity value" is 540 by "4 mPa·s $\times$ 30 parts by mass + 6 mPa·s $\times$ 70 parts by mass".

Generally, the lower the molecular weight, the lower the viscosity value. In addition, when the molecular weight is low, that is, when the viscosity value is low, the hard capsule has better solubility, but on the other hand, it tends to break more easily.

Therefore, for orally administered pharmaceuticals, where the solubility is usually important, the "hydroxypropyl methylcellulose viscosity value" is preferably in the range of 300 to 960. On the other hand, for inhalation pharmaceuticals and food applications, where resistance to cracking is important, the "hydroxypropyl methylcellulose viscosity value" is preferably in the range of 500 to 1500.

The content of the cellulose compound in the hard capsule shell is the remaining amount after subtracting the total amount of shell components other than the cellulose compound such as gelling agent, gelling aid, light-shielding agent, plasticizer, lubricant, metal sequestering agent, colorant, and light-shielding agent, which will be described later. However, the shell components other than the cellulose compound do not include moisture.

The cellulose compound can accelerate gelation during shell formation by adding (1) the gelling agent, or (2) the gelling agent and the gelling aid.

Examples of the gelling agents may include carrageenan, pectin, gellan gum, and the like, which are capable of gelling a hard capsule preparation solution. These can be used as one type alone or in any combination of two or more types.

Among the above gelling agents, the carrageenan is the most suitable gelling agent because it has high gel strength and has excellent gelation property when used in a small amount in the presence of specific ions. Three types of carrageenan are generally known: kappa-carrageenan, iota-carrageenan and lambda-carrageenan. In the present invention, the kappa and iota-carrageenans, which have gelling ability resulting in relatively high hardness, can be used suitably, and kappa-carrageenan can be used more suitably. The pectin can be classified into LM pectin and HM pectin according to the degree of esterification, and the gellan gum can be classified into acylated gellan gum (native gellan gum) and deacylated gellan gum according to the presence or absence of acylation, but either of them can be used in the present invention without any distinction.

The content of the gelling agent in the shell of the hard capsule is not limited as long as the shell of the hard capsule can be molded by a cooling gel method. The content of the gelling agent may be 0.05 to 10 mass%, preferably 0.1 to 9.5 mass%, more preferably 0.2 to 9 mass%, and even more preferably 0.3 to 8 mass%, when the total of the shell components of the hard capsule excluding moisture is set to 100 mass%.

The gelling aid can be selected according to a type of the gelling agent to be used. The gelling aid has an effect of accelerating the gelling of the gelling agent. Alternatively, it may contribute to the acceleration of gelation by directly acting on the cellulose compound to raise or lower its gelation temperature or cloud point temperature. When the carrageenan is used as the gelling agent, examples of the gelling aids that can be used in combination with the gelling agent, for kappa-carrageenan, may include compounds capable of producing one or more of sodium ions, potassium ions, ammonium ions and calcium ions in an aqueous solution, such as sodium chloride, potassium chloride, potassium phosphate, ammonium chloride, ammonium acetate and calcium chloride. Preferably, it is a compound that produces the sodium ions, potassium ions or calcium ions in the aqueous solution. For iota-carrageenan, examples may include compounds capable of donating calcium ions in water, such as calcium chloride. When the gellan gum is used as the gelling agent, examples of the gelling aids that can be used in combination with the gelling agent may include compounds capable of donating one type or two or more types of ions selected from sodium ions, potassium ions, calcium ions and magnesium ions in water, such as sodium chloride, potassium chloride, calcium chloride and magnesium sulfate. In addition, an organic acid or a water-soluble salt thereof, such as citric acid or sodium citrate, may also be used.

In addition, the calcium carbonate used as the light-shielding agent in the present invention also produces calcium ions slightly in the hard capsule preparation solution, so that when the gellan gum and/or iota-carrageenan are used as the gelling agent, the hard capsule preparation solution can be gelled without adding the gelling aid.

The content of the gelling aid in the shell of the hard capsule may be set according to the content of the gelling agent. The content of the gelling aid may be in the range greater than 0 and less than or equal to 2.2 mass%, preferably 0.1 to 2.1 mass%, more preferably 0.2 to 1.9 mass%, and even more preferably 0.3 to 1.6 mass%, when the total of the shell components of the hard capsule excluding moisture is set to 100 mass%. When the gellan gum and/or iota-carrageenan are used as the gelling agents, the content of the gelation aid may be in the range of 0 to 2.2 mass%, preferably 0.1 to 2.1 mass%, more preferably 0.2 to 1.9 mass%, and even more preferably 0.3 to 1.6 mass%, when the total of the shell components of the hard capsule excluding moisture is set to 100 mass%.

When the hydroxypropyl methylcellulose is used as the cellulose compound, the carrageenan, especially the kappa-carrageenan, may be suitably used as the gelling agent in combination, and the potassium chloride may be suitably used as the gelling aid in combination with the gelling agent.

The light-shielding agent contains calcium carbonate. The calcium carbonate has two types: one is produced by pulverizing limestone, and the other is produced industrially. The calcium carbonate produced by pulverizing limestone is called heavy calcium carbonate. The calcium carbonate produced industrially is called light calcium carbonate, precipitated calcium carbonate, or synthetic calcium carbonate.

Either type of the calcium carbonate may be used for the hard capsule shell, but the heavy calcium carbonate is preferred.

The particle diameter of the calcium carbonate can be expressed by median diameter. Preferably, Dv50, which is a median diameter by volume, can be used. The median diameter of a particle can be determined by Coulter counter, laser diffraction scattering, or the like.

Definitions of laser diffraction method and particle diameter, average particle diameter, and volume fraction obtained by the same method are in accordance with JIS Z 8825.

Regarding the Dv50 of the calcium carbonate, as shown in FIG. 1A, when a transmittance of the shell having a thickness of 100 $\mu$m and containing 7 mass% calcium carbonate relative to the shell component is measured using light at a wavelength of 650 nm, the Dv50 is preferably 0.5 $\mu$m to 7 $\mu$m to achieve a transmittance of 40% or less, the Dv50 is preferably 0.6 $\mu$m to 5 $\mu$m to achieve a transmittance of 30% or less, and the Dv50 is preferably 0.9 $\mu$m to 3.8 $\mu$m to achieve a transmittance of 20% or less. As shown in FIG. 1B, when a transmittance of the shell having a thickness of 100 $\mu$m and containing 7 mass% calcium carbonate relative to the shell component is measured using light at a wavelength of 420 nm, the Dv50 is preferably 0.4 $\mu$m to 6 $\mu$m to achieve a transmittance of 20% or less, and the Dv50 is preferably 0.7 $\mu$m to 3.4 $\mu$m to achieve a transmittance of 10% or less. Therefore, the Dv50 of the calcium carbonate is preferably 0.7 $\mu$m to 3.8 $\mu$m. Here, when the thickness of the shell is 100 $\mu$m, the measured value of the thickness of the shell may be 100 $\mu$m, or the measured value of the thickness of the shell may be converted to 100 $\mu$m. A definition of the transmittance is described below.

As a commercially available heavy calcium carbonate, Precipitated Calcium Carbonate A (BIHOKU FUNKA KOGYO CO., LTD., Dv50: 2.16$\mu$m, * The name is Precipitated Calcium Carbonate, but the manufacturer's label is Heavy Calcium Carbonate), Aragen (CALFINE Co., Ltd., Dv50: 5.05$\mu$m), CALTEX 5 (MARUO CALCIUM CO., LTD., Dv50: 1.82$\mu$m), ACE-30 (CALFINE Co., Ltd., Dv50: 1.89$\mu$m), ACE-35 (CALFINE Co., Ltd., Dv50: 2.04$\mu$m), CALMIGEN A (BIHOKU FUNKA KOGYO CO., LTD., Dv50: 2.12$\mu$m), NANOX#25A (MARUO CALCIUM CO., LTD., Dv50: 3.02$\mu$m), NANOX#30 (MARUO CALCIUM CO., LTD., Dv50: 2.21$\mu$m), CALCY-F (SANKYO SEIFUN CO., LTD., Dv50: 2.44$\mu$m), Kristone-SS food additive (CALFINE Co., Ltd., Dv50: 3.02$\mu$m) and the like can be used.

As the precipitated calcium carbonate, Calpin F (YABASHI INDUSTRIES CO., LTD., Dv50: 2.63$\mu$m), Calessen I (Shiraishi Kogyo Kaisha, Ltd., Dv50: 0.39$\mu$m), CAL·ACE-ST (MARUO CALCIUM CO., LTD., Dv50: 0.51$\mu$m) and the like can be used.

The above Dv50 value is the median diameter by volume and indicates the measured value by the laser diffraction method described in the JIS Z 8825.

The content of the light-shielding agent in the shell of the hard capsule is, for example, 3 to 20 mass% when the total of the shell components of the hard capsule excluding moisture is set to 100 mass%. Preferably, it is 6 to 20 mass%.

The shell of the hard capsule may contain, if necessary, a plasticizer, lubricant, metal sequestering agent, colorant, light-shielding agent, residual moisture (also referred to simply as moisture), or the like as components of the shell of the hard capsule.

The plasticizer is not particularly limited as long as it can be used in pharmaceutical or food compositions and may include, for example, dioctyl adipate, polyester adipate, epoxidized soybean oil, epoxyhexahydrophthalic acid diester, kaolin, triethyl citrate, glycerin, glycerin fatty acid ester, sesame oil, dimethyl polysiloxane-silicon dioxide mixture, D-sorbitol, medium-chain fatty acid triglyceride, corn starch-derived sugar alcohol liquid, triacetin, concentrated glycerin, castor oil, phytosterol, diethyl phthalate, dioctyl phthalate, dibutyl phthalate, butyl phthalyl butyl glycolate, propylene glycol, polyoxyethylene (105) polyoxypropylene (5) glycol, polysorbate 80, macrogol 1500, macrogol 400, macrogol 4000, macrogol 600, macrogol 6000, isopropyl myristate, cotton seed oil-soybean oil mixture, glycerin monostearate, isopropyl linoleate, and the like. When the plasticizer is used, it may be used in a range of 15 mass% or less, when the total of the shell components of the hard capsule excluding moisture is set to 100 mass%. It may be added in a range of preferably 13 mass% or less, more preferably 11 mass% or less, and even more preferably 8 mass% or less.

Examples of the metal sequestering agent may include ethylenediaminetetraacetic acid, acetic acid, boric acid, citric acid, gluconic acid, lactic acid, phosphoric acid, tartaric acid, or salts thereof, metaphosphate, dihydroxyethylglycine, lecithin, $\beta$-cyclodextrin, and combinations thereof.

The lubricant is not particularly limited as long as it can be used in the pharmaceutical or food compositions. Examples thereof may include calcium stearate, magnesium stearate, sodium stearyl fumarate, carnauba wax, starch, sucrose fatty acid ester, light anhydrous silicic acid, macrogol, talc, hydrogenated vegetable oil, and the like.

The colorant and light-shielding agent are not particularly limited as long as they can be used in the pharmaceutical or food compositions. Examples of the colorant may include powdered gambir tannin, turmeric extract, methylrosaniline chloride, yellow iron oxide, yellow iron sesquioxide, Opaspray K-1-24904, orange essence, brown iron oxide, carbon black, caramel, carmine, carotene liquid, $\beta$-carotene, photosensitizer 201, licorice extract, gold leaf, Sasa veitchii extract, black iron oxide, light anhydrous silicic acid, Daemonorops draco (kekketsu), zinc oxide, titanium oxide, iron sesquioxide, disazo yellow, Food Blue No. 1 and its aluminum lake, Food Blue No. 2 and its aluminum lake, Food Yellow No. 4 and its aluminum lake, Food Yellow No. 5 and its aluminum lake, Food Green No. 3 and its aluminum lake, Food Red No. 2 and its aluminum lake, Food Red No. 3 and its aluminum lake, Food Red No. 102 and its aluminum lake, Food Red No. 104 and its aluminum lake, Food Red No. 105 and its aluminum lake, Food Red No. 106 and its aluminum lake, sodium hydroxide, talc, sodium copper chlorophyllin, copper chlorophyll, powdered hull-less barley green tea extract, hull-less barley green tea extract, phenol red, sodium fluorescein, d-borneol, malachite green, octyldodecyl myristate, methylene blue, medicinal carbon, riboflavin butyrate, riboflavin, powdered green tea, manganese ammonium phosphate, riboflavin sodium phosphate, rose oil, turmeric oleoresin, chlorophyll, carminic acid, Food Red No. 40 and its aluminum lake, water-soluble annatto, sodium iron chlorophyllin, dunaliella carotene, paprika color, carrot carotene, potassium norbixin, sodium norbixin, palm oil carotene, beat red, grape skin color, black currant color, monascus color, Carthamus red color, Carthamus yellow color, marigold color, riboflavin sodium phosphate, madder color, alkanet color, aluminum, sweet potato carotene, shrimp color, krill color, orange color, cacao color, cacao carbon black, Japanese persimmon color, crab color, carob germ color, fish scale foil, silver, kusagi (Clerodendrum trichotomum) color, gardenia blue, gardenia red, gardenia yellow, kooroo color, chlorophyll, kaoliang color, bone carbon black, bamboo grass color, Shea nut color, lithospermum root color, sandalwood red, vegetable carbon black, sappan wood color, spirulina color, onion color, tamarind color, corn color, tomato color, peanut color, phaffia color, pecan nut color, monascus yellow, powdered annatto, haematococcus algae color, purple sweet potato color, purple corn color, purple yam color, vegetable oil soot color, lac color, rutin, enju (Styphnolobium japonicum) extract, buckwheat whole-plant extract, logwood color, red cabbage color, red rice color, red radish color, adzuki bean color, sweet hydrangea leaf extract, sepia color, uguisukagura (Lonicera gracilipes) color, elderberry color, olive tea, cowberry color, gooseberry color, cranberry color, salmonberry color, strawberry color, dark sweet cherry color, cherry color, thimbleberry color, deberry color, pineapple juice, huckleberry color, grape juice color, black currant color, blackberry color, plum color, blueberry color, berry juice, boysenberry color, whortleberry color, mulberry color, morello cherry color, raspberry color, red currant color, lemon juice, loganberry color, powdered chlorella, cocoa, saffron color, perilla color, chicory color, laver color, hibiscus color, malt extract, paprika powder, beet red juice, carrot juice, and the like.

Examples of the light-shielding agent may include titanium oxide, iron sesquioxide, yellow iron sesquioxide, black iron oxide, Food Blue No. 1 aluminium lake, Food Blue No. 2 aluminium lake, Food Yellow No. 4 aluminium lake, Food Yellow No. 5 aluminium lake, Food Green No. 3 aluminium lake, Food Red No. 2 aluminium lake, Food Red No. 3 aluminum lake, Food Red No. 102 aluminium lake, Food Red No. 104 aluminium lake, Food Red No. 105 aluminium lake, Food Red No. 106 aluminium lake, Food Red No. 40 aluminium lake.

It is preferable that the capsule shell after preparation usually contains a few percent residual moisture. Usually, when the capsules after molding is dried in the range of 30 to 100°C, the residual moisture content settles to a specific saturated residual moisture value corresponding to a solid content of the capsules and their composition. Naturally, when the drying treatment is performed at a higher temperature, the residual moisture content settles to the saturated moisture value in a shorter time. The residual moisture depends on the environmental humidity during capsule storage, and changes almost reversibly. Specifically, the saturated moisture value after sufficient drying treatment at 30 to 100°C and further storage at a constant temperature and relative humidity for several days settles to a constant value. In the present invention, the saturated moisture value after storage at room temperature and the relative humidity of 43% for several days is used.

It is rather preferable that a small amount of the residual moisture is included to maintain resistance to cracking. The saturation moisture value of the residual moisture at room temperature and 43% relative humidity is preferably at least 1% or more, more preferably 2% or more, and even more preferably 3% or more of the total mass of the capsule shell. On the other hand, if the residual moisture is too much, it may react with a drug filled inside when stored for a long period of time, so the residual moisture value is preferably 8% or less, and even more preferably 6% or less.

A saturated residual moisture content can be expressed as the water content by loss on drying and can be measured as follows.

<Method for Measuring Water Content in Capsule Shell by Loss on Drying Method>

A sample (hard capsule, or film) is placed in a desiccator which contains a saturated aqueous solution of potassium carbonate and thereby has an atmosphere of constant humidity, seal, and condition the humidity at 25°C for 1 week. In the presence of the saturated aqueous solution of potassium carbonate, an atmosphere having a relative humidity of about 43% can be obtained. A mass of the sample after conditioning the humidity (wet mass) is measured, and then the sample is heated and dried at 105°C for 2 hours, and the mass of the sample (dry mass) is measured again. From a difference between the mass before drying (wet mass) and the mass after drying (dry mass), the percentage of moisture content (water content) decreased by the heating and drying at 105°C for 2 hours is calculated according to the following equation.

Equation 1

$$\text{water content (\%)} = \frac{\text{(wet mass of sample)} - \text{(dry mass of sample)}}{\text{wet mass of sample}} \times 100$$

The shell of the hard capsule described above has light-shielding ability. The light-shielding ability can be evaluated by a transmittance. The transmittance can be calculated according to the following method.

<Evaluation of light-shielding ability>

When evaluating permeability of the hard capsules, it is important to compare thicknesses of the test shells by matching the thicknesses. Therefore, for example, for the permeability of the hard capsule, a cast film is prepared by a casting method using a preparation solution having the same component composition as each component composition of the hard capsule preparation solution, and then the permeability is evaluated using the cast film.

A method for preparing the cast film is as follows.

When the cast film is prepared by the cooling gel method, a metallic applicator is placed on a glass surface or PET film kept at room temperature, and the preparation solution of approximately 50 to 60°C is poured onto the glass surface or PET film and the applicator is moved at a constant speed to prepare a uniform wet film such that the film thickness after drying is 100 $\mu$m. Thereafter, the wet film can be dried at room temperature to about 30°C for about 10 hours to obtain the cast film.

When preparing the cast film by a heating gel method, the metallic applicator is placed on the glass surface or PET film kept at 60°C, and the preparation solution of room temperature is poured onto the glass surface or PET film and the applicator is moved at a constant speed to prepare a uniform wet film such that the film thickness after drying is 100 $\mu$m. Thereafter, the wet film can be dried at about 60°C for about 1 hour, and further dried at room temperature for about 10 hours to obtain the cast film. In order to obtain the film having the uniform film thickness of 100 $\mu$m, applicators having a gap of 0.4 mm to 1.5 mm may be used as appropriate.

The light-shielding ability can be evaluated, for example, by measuring an intensity of transmitted light transmitted through an object to be measured using a UV-visible spectrophotometer (e.g., UV-1850, SHIMADZU CORPORATION), and calculating the transmittance. The transmittance can be calculated by, for example, cutting the cast film prepared by the above method into strips of 10 mm×20 mm, placing the cast film vertically to a light path of the UV-visible spectrophotometer so as to block the light path, irradiating the cast film with light having a wavelength of 650 nm or 420 nm, measuring an intensity of transmitted light transmitted through the cast film and an intensity of transmitted light of a blank when the cast film is not placed, and dividing the intensity of the transmitted light transmitted through the cast film by the intensity of the transmitted light of the blank. The transmittance and light-shielding ability are inversely related, that is, the lower the transmittance, the higher the light-shielding ability. The calculated transmittance may be corrected by the thickness of the shell. The corrected transmittance can be calculated by taking a theoretical value of the thickness of the shell as 100 $\mu$m, multiplying a value obtained by dividing the theoretical value by the measured value ($\mu$m) of the thickness of the shell by a value obtained by converting the calculated transmittance into the absorbance, and then converting the value back into transmittance.

The shell of the hard capsule described above has a predetermined strength. The strength can be evaluated according to the following method.

<Evaluation of strength>

For the cast film prepared by the above method, a dumbbell-shaped test piece made by cutting the cast film into a dumbbell shape (as specified in JIS K-7161-2-1BA) of 5 mm × 75 mm, for example, is prepared. This dumbbell-shaped test piece can be used to perform a tensile test using, for example, a compact table-top testing machine (EZ-LX, SHIMADZU CORPORATION). Specifically, both ends of the film are set in a holder (gap length 59 mm) and pulled at a tensile speed of 50 mm/min to obtain a film elongation and stress generated in the film (tensile stress) - elongation rate (strain) curve. From an integral value of the tensile stress-elongation rate curve up to a breaking point, toughness (MJ/m$^3$) can be obtained as an index of the resistance to cracking (Reference: Aqueous Polymeric Coating For Pharmaceutical Dosage Forms, 4th edition, CRC Press, 2017, Chapter 4).

The above strength is preferably maintained under normal conditions of use. For this reason, it is preferable that, for example, the dumbbell-shaped test piece which has been prepared and cut into the dumbbell shape is conditioned with humidity at 25°C and a relative humidity of 22% (using a saturated salt solution of potassium acetate) for at least 1 week, and then the tensile test is performed under the same temperature and humidity environment as the condition for conditioning humidity to evaluate a mechanical strength.

The toughness, which is the index of the resistance to cracking, is preferably 2.0 MJ/m$^3$ or higher. When the toughness is less than 0.6 MJ/m$^3$, the shell is significantly prone to cracking even in normal handling.

2. Hard capsule preparation solution

The hard capsule preparation solution (hereinafter, also referred to simply as "preparation solution") for preparing the hard capsule of this embodiment contains an aqueous solvent and the shell components described in the above section 1. The aqueous solvent is preferably water, ethanol, and a mixture thereof, and more preferably the water.

The content of the above shell component contained in the preparation solution is not limited as long as the shell of the hard capsule can be molded by the cooling gel method, and the content of each component in the hard capsule after preparation is equal to the content in the above hard capsule. That is, in the preparation solution, the content of the shell component contained in the preparation solution is not limited as long as the content of each component in the hard capsule after preparation is equal to the content in the above hard capsule when the total of the components excluding the aqueous solvent of the preparation solution is set to 100 mass%. For example, the following concentrations can be listed as final concentrations in the preparation solution. The final concentration refers to a concentration in a final solution, that is, a concentration in a solution that is actually used in the preparation of the capsule.

For the cellulose compound, the content is 10 to 30 mass%, preferably 12 to 20 mass%, and more preferably 14 to 18 mass%; for the light-shielding agent, the content is 0.6 to 10 mass%, preferably 1 to 6 mass%, and more preferably 2 to 4 mass%. When components other than a base and a hardness improver are contained, for the gelling agent, the content may be 0.005 to 0.5 mass%, preferably 0.01 to 0.45 mass%, and more preferably 0.015 to 0.4 mass%. When the gelling aid is used, its concentration may be 0.5 mass% or less, 0.02 to 0.5 mass%, preferably 0.03 to 0.40 mass%, and more preferably 0.04 to 0.35 mass%. When a lubricant, colorant, light-shielding agent, metal sequestering agents, flavor, or the like are contained, their content may be set appropriately in a range of 0.5 mass% or less, respectively.

The total content of the shell components other than the aqueous solvent in the said preparation solution may be in a range of 10 to 30 mass%, preferably 12 to 25 mass%, and more preferably 14 to 20 mass%, when the total of the preparation solution is set to 100 mass%.

3. Hard capsule preparation method

A preparation method of hard capsule preparation solution (also called immersion solution) is not particularly limited. For example, a method in which the water-soluble cellulose compound is dispersed in purified water heated to about 70-80 °C, wherein the gelling agent or the gelling aid is dissolved as necessary, and then cooled to a desired temperature for the immersion solution (usually 35 to 60°C, more preferably 40 to 60°C) to dissolve the water-soluble cellulose compound to prepare a uniform capsule preparation solution (immersion solution); a method in which the water-soluble cellulose compound is dispersed in hot water of about 70 to 80°C, the solution is once cooled to dissolve the water-soluble cellulose compound, a gelling agent or a gelling aid is then added as necessary and dissolved, the solution is heated again to adjust the temperature to about 30 to 50°C to prepare a uniform capsule preparation solution (immersion solution), and the solution is then adjusted to the desired temperature for the immersion solution; and the like may be used without limitation. Furthermore, in a molding method by thermal gelation described below, a method in which the water-soluble cellulose compound is dispersed in hot water of about 70 to 90°C without adding the gelling agent and gelling aid, and then cooled once to near or below room temperature to dissolve the water-soluble cellulose compound to prepare the hard capsule preparation solution may be used.

A viscosity of the capsule preparation solution is not particularly limited. Preferably, the viscosity of the capsule preparation solution may be prepared so that the viscosity of the capsule preparation solution is 100 to 20000 mPa·s, preferably 300 to 10000 mPa·s, under a temperature (temperature of immersion solution) condition employed during immersion of a pin for molding a capsule (30 to 80°C, preferably 40 to 60°C). Usually, a solvent content of the capsule preparation solution may be 60 to 90 mass%, preferably 70 to 85 mass%. The total content of the hard capsule shell components other than the solvent in the capsule preparation solution may be 10 to 40 mass%, preferably 15 to 30%.

The viscosity specified in the present invention refers to a viscosity measured in a B-type rotational viscometer at a predetermined temperature, at a rotation speed of 60rpm and a measurement time of 1 minute, using rotor number 2 when the viscosity is less than 500 mPa·s, rotor number 3 when the viscosity is 500 mPa·s or more and less than 2000 mPa·s, and rotor number 4 when the viscosity is 2000 mPa·s or more.

Concentrations of each component contained in the capsule preparation solution are described below.

A hard capsule preparation (molding) method is not particularly limited as long as it includes a step of preparing the capsule using the capsule preparation solution according to the present invention. The hard capsule is generally formed by immersing the mold pin serving as a mold for the capsule in an aqueous solution in which a capsule shell material is dissolved, and curing and drying the coating film adhering to the mold pin when it is pulled up to obtain a desired capsule shape and thickness (dipping method). Specifically, the hard capsule preparation method includes a step of preparing the capsule preparation solution by the method described above, or preparing the capsule preparation solution through, for example, purchase thereof and a step of immersing the pin for molding the capsule in the capsule preparation solution, pulling up the pin, gelling the solution adhering to the pin for molding the capsule, and drying the gelled shell at 20 to 80°C. In some cases, the molding may be performed by increasing the viscosity by cooling and drying without going through the gelling step.

More specifically, the hard capsule prepared by the cooling gel method may be prepared through the following molding steps.

(1) A step of immersing the pin for molding the capsule in the capsule preparation solution (immersion solution) containing the cellulose compound (and, if necessary, the gelling agent or gelling aid) (immersion step);
(2) a step of pulling up the pin for molding the capsule from the capsule preparation solution (immersion solution) and gelling the capsule preparation solution adhering to the outer surface of the pin (gelling step);
(3) a step of drying the gelled capsule film (gelled shell) formed to coat the outer surface of the pin for molding the capsule (drying step); and
(4) a step of demolding the dried capsule film (shell) from the pin for molding the capsule (demolding step).
If necessary, the following heating step may be performed after the step (4).
(5) A step of treating the gelled capsule film (gelled shell) with heat at 30 to 150°C after the gelling step (2), before or after or simultaneously with the drying step (3), or after the demolding step (4) (heating step).

When a solution not containing the gelling agent such as the carrageenan is used as the capsule preparation solution (immersion solution), the gelling step (2) may be performed by using the pin for molding the capsule heated to 60°C or more by utilizing the fact that the water-soluble cellulose compound itself gels at a temperature of 60°C or more (heating gel method). Specifically, in the immersion step (1), the pin for molding the capsule heated to 60 to 150°C, preferably 60 to 120°C, and more preferably 70 to 90°C according to the temperature of the capsule preparation solution (immersion solution) adjusted to a constant temperature of 25 to 50°C, preferably 35 to 45°C is immersed in the capsule preparation solution, and then, in the gelling step (2), the pin for molding the capsule is pulled up from the capsule preparation solution (immersion solution) to gel capsule preparation solution adhering to the outer surface of the pin.

On the other hand, when a solution containing the gelling agent such as the carrageenan is used as the capsule preparation solution (immersion solution), the gelling step (2) may be performed by cooling the capsule preparation solution adhering to the outer surface of the pin for molding the capsule (cooling gel method) by setting the temperature around the capsule manufacturing machine to usually 35°C or less, preferably 30°C or less, preferably at room temperature by utilizing the fact that the capsule preparation solution gels at a temperature of 50°C or less. Specifically, in the immersion step (1), the pin for molding the capsule adjusted to 10 to 30°C, preferably 13 to 28°C, and more preferably 15 to 25°C according to the temperature of the capsule preparation solution (immersion solution) adjusted to a constant temperature of 35 to 60°C, preferably 40 to 60°C is immersed in the capsule preparation solution, and then, in the gelling step (2), the pin for molding the capsule is pulled up from the capsule preparation solution (immersion solution) to gel the capsule preparation solution adhering to the outer surface of the pin.

The drying step (3) may be performed at room temperature. It is usually performed by blowing air at room temperature. The demolding step (4) is performed by removing the dried capsule film formed on the surface of the pin for molding the capsule from the pin for molding the capsule.

The heating step (5), which is an optional step, may be performed after the gelling step (2), that is, after the capsule preparation solution is gelled (solidified). The heating treatment may be performed at any stage after the gelling step (2), before or after the drying step (3), or the heating and drying may be performed at the same time. Further, the heating treatment may be performed after the demolding step (4). Preferably, after the gelling step (2), the gelled capsule film is subjected to the drying step at room temperature, and after drying or at the half-dried stage, the heating treatment is performed. A heating temperature is not limited as long as it is in a range of 30 to 150°C, but preferably in a range of 40 to 100°C, more preferably in a range of 50 to 80°C. The heating treatment may be performed by blowing air, usually at 30 to 150°C.

The capsule film thus prepared, after being cut and adjusted to a predetermined length, may be provided as the hard capsule with the body portion and cap portion fitted or not fitted into a pair.

The thickness of the shell of the hard capsule is usually in a range of 50 to 200 $\mu$m. In particular, the thickness of a side wall portion of the capsule is generally 70 to 150 $\mu$m, more preferably 80 to 120 $\mu$m for capsules currently commercially available. The hard capsule comes in a variety of sizes, including No. 00, No. 0, No. 1, No. 2, No. 3, No. 4, No. 5, and the like, but any size of the hard capsule may be used in the present invention. The capsule shell may also be obtained by a solidification method that relies solely on moisture evaporation and drying from the capsule preparation solution without particularly accompanying a gelation phenomenon.

4. Filling of content into hard capsule and application

A method of filling the hard capsule with a content is not particularly restricted.

The content may be filled into the hard capsule using a known capsule filling machine described in Japanese Unexamined Patent Application No. 2007-144014, Japanese Unexamined Patent Application No. 2000-226097, and others, for example, a fully-automatic capsule filling machine (model name: LIQFIL super 80/150, manufactured by Qualicaps Co., Ltd.), a capsule filling and sealing machine (model name: LIQFIL super FS, manufactured by Qualicaps Co., Ltd.) or the like.

In the above filling method, the temporary and main binding of the hard capsule is secured by a locking mechanism as shown in US Patent No. 3508678, US Patent No. 3823843, US Patent No. 4040536, US Patent No. 4822618, US Patent No. 5769267, and others. The strength of the hard capsule is also important to stably maintain the locking mechanism as described above.

In addition to the above lock mechanism by fitting the cap to the body, a fitted portion may be sealed with a band seal as described in Japanese Unexamined Patent Application No. 2005-187412 or Japanese Unexamined Patent Application No. 2009-504630 to prevent malicious opening and contamination of foreign matter, and to ensure prevention of leakage of liquid fillings.

Application of the hard capsule of the present invention is not particularly limited. Preferably, an oral formulation, an inhalation formulation and the like may be included.

The oral formulation is desirable to dissolve quickly in a stomach or intestine. The oral formulation may also be an enteric capsule with a coating of enteric base material added to the surface of the capsule shell in order to allow the capsule shell to dissolve in the intestine and release the drug in the intestine. Oral formulations may also be an enteric capsule formed by using an enteric base material in whole or in part of the capsule shell itself. The enteric capsule is not particularly limited as long as it has a property of being dissolved in the intestine without being dissolved in the stomach, but refers to, for example, one that is virtually insoluble in dilute hydrochloric acid solution at pH 1.2 (first fluid of Japanese Pharmacopoeia) for more than 2 hours and is dissolved in buffer solution at pH 6.8 (second fluid of Japanese Pharmacopoeia).

The drug may also be sustained-released from the hard capsule. In the case where the drug is gradually released, the capsule shell surface may be coated with a sustained-release coating.

The inhalation formulation is a hard capsule containing a single dose of drug and attached to a device such as those disclosed in US Patent No.4069819, US Patent No. 4210140, US Patent No. 7669596, US Patent Application Publication No. 2010-0300440, and others. By puncturing the capsule with a small pin or breaking the capsule, the drug inside can be inhaled at an appropriate flow rate.

The content of the hard capsule is not particularly limited and may include, without limitation, human or animal pharmaceuticals, quasi-drugs, cosmetics, and foods.

The form of the contents is also not limited. For example, it may be in a liquid form, a gel form, a powder form, a granular form, a tablet form, a pellet form, or a mixed form (hybrid form) thereof.

In the case of the pharmaceuticals, the content of the hard capsule may include one or more drug components selected from, for example, nutritional and tonic health agent, antipyretic analgesic antiinflammatory agent, psychotropic, anxiolytic, antidepressant, hypnotic, antispasmodic, central nervous system agent, brain metabolic stimulant, cerebral circulation improver, antiepileptic drug, sympathomimetic drug, gastrointestinal agent, antacid, antiulcer drug, antitussive and expectorant combination, antiemetic, respiratory stimulant, bronchodilator, allergy medicine, dental and oral agent, antihistamine, cardiotonic agent, antiarrhythmic drug, diuretic, hypotensive agent, vasoconstrictor, coronary vasodilator, peripheral vasodilator, lipid-lowering agent, cholagogue, antibiotic, chemotherapeutic agent, antidiabetic drug, drug for osteoporosis, antirheumatic drug, skeletal muscle relaxant, spasmolytic, hormone preparation, alkaloid narcotic, sulfonamide, gout suppressant, anticoagulant, antineoplastic drug, and the like. These medicinal components are not particularly limited and may include a wide range of known components, but specifically, each of the components described in paragraphs [0055] to [0060] of the WO2006/070578 pamphlet may be exemplified. WO2006/070578 is incorporated herein by reference.

In the case of the foods, for example, functional components such as docosahexaenoic acid, eicosapentaenoic acid, $\alpha$-lipoic acid, royal jelly, isoflavones, agaricus, acerola, aloe, aloe vera, turmeric, L-carnitine, oligosaccharide, cacao, catechin, capsaicin, chamomile, agar, tocopherol, linolenic acid, xylitol, chitosan, GABA, citric acid, chlorella, glucosamine, Panax ginseng root, coenzyme Q10, brown sugar, collagen, chondroitin, Polyporaceaem, squalene, stevia, ceramide, taurine, saponin, lecithin, dextrin, Houttuynia cordata, niacin, Bacillus subtilis var natto, bittern, lactobacilli, saw palmetto, honey, Coix, plum extract, pantothenic acid, hyaluronic acid, vitamin A, vitamin K, vitamin C, vitamin D, vitamin B1, vitamin B2, vitamin B6, vitamin B12, quercetin, protein, propolis, mulukhiya, folic acid, lycopene, linoleic acid, rutin, reishi, and the like may be included. However, it is not limited to these.

Examples

Hereinafter, the embodiment of the present invention will be described in more detail with reference to examples.
However, the present invention should not be construed as limited to the examples.

1. Experimental Example 1: Measurement of transmittance and strength of a film-like capsule shell

When evaluating transmittance and strength of the hard capsules, a measured value changes depending on the thickness of the capsule shell, especially the shell thickness of the capsule body into which a metal indenter is pressed. For the evaluation of transmittance and strength, it is important to match the thicknesses of the test shells for comparison. Therefore, for the evaluation of transmittance and strength dependent on the component composition of the hard capsule, instead of the hard capsule molded by the dipping method, a film having the same component composition as that of the hard capsule was prepared by the casting method for each component composition of the hard capsule shell, and the transmittance and strength were evaluated using this film. In the following, instead of the hard capsule molded by the dipping method, a film having the same component composition as each component composition of the hard capsule is prepared and evaluated, and the film has excellent uniformity of thickness, excellent reproducibility of evaluation, and reflects well the transmittance and strength as the capsule shell.

1-1. Hard capsule preparation solution

Compositions of each hard capsule prepared by adding calcium carbonate as a standard, an example, and a comparative example are shown in Tables 2-1 and 2-2. As for HPMC, jelly was prepared by changing the blend ratio of HPMC shown in i, ii and iii below.

    i. substitution type; 2910, Shin-Etsu Chemical Co., Ltd., viscosity value; 6
    ii. substitution type; 2910, Lotte Fine Chemical Co., Ltd., viscosity value; 4.5
    iii. substitution type; 2208, Shin-Etsu Chemical Co., Ltd., viscosity value; 4

The HPMC having a viscosity value of 530 was prepared by mixing the HPMC having a viscosity value of 6, the HPMC having a viscosity value of 4.5 and the HPMC having a viscosity value of 4 at 6:2:2. The HPMC having a viscosity value of 570 was prepared by mixing the HPMC having a viscosity value of 6 and the HPMC having a viscosity value of 4.5 at 8:2.
As a heavy calcium carbonate, Precipitated Calcium Carbonate A (BIHOKU FUNKA KOGYO CO., LTD., Dv50: 2.16μm), Aragen (CALFINE Co., Ltd., Dv50: 5.05μm), CALTEX 5 (MARUO CALCIUM CO., LTD., Dv50: 1.82μm), ACE-35 (CALFINE Co., Ltd., Dv50: 2.04μm), CALMIGEN A (BIHOKU FUNKA KOGYO CO., LTD., Dv50: 2.12μm), NANOX#25A (MARUO CALCIUM CO., LTD., Dv50: 3.02μm), NANOX#30 (MARUO CALCIUM CO., LTD., Dv50: 2.21μm), and CALCY-F (SANKYO SEIFUN CO., LTD., Dv50: 2.44μm) were used.
As a precipitated calcium carbonate, Calpin F (YABASHI INDUSTRIES CO., LTD., Dv50: 2.63μm), Calessen I (Shiraishi Kogyo Kaisha, Ltd., Dv50: 0.39μm), CAL·ACE-ST (MARUO CALCIUM CO., LTD., Dv50: 0.51μm) were used. The above Dv50 value is a median diameter by volume and indicates the measured value by the laser diffraction method described in the JIS Z 8825.
Titanium oxide was added to the standard as a light-shielding agent.
A preparation solution for hard capsule prepared by a cooling gel method was prepared according to the following method. Carrageenan and potassium chloride were added to pure water, stirred and dispersed, and it was confirmed that they were dissolved after a temperature of the mixture was raised to 80°C. While maintaining the temperature of the solution at 80°C, the HPMC was added and dispersed and allowed to stand for 30 minutes, and bubbles were removed by vacuum debubbling. Subsequently, the temperature was lowered to 50 to 60°C while stirring with a three-one motor, stirred with the three-one motor for 1 hour, and then added with calcium carbonate and stirred to disperse the calcium carbonate sufficiently and uniformly to prepare a jelly-like capsule preparation solution. The calcium carbonate was prepared in advance as an 11 to 22 mass% of water dispersion, and homogenized (9,000 rpm, 15 minutes) using a homogenizer (manufactured by IKA; generator used: S25N-25F) before being added.
A preparation solution for hard capsule prepared by a heating gel method was prepared according to the following method. After raising a temperature of pure water to 80°C, the HPMC was added and dispersed in the pure water, allowed to stand for 30 minutes, and then bubbles were removed by vacuum debubbling. Subsequently, the temperature was lowered to room temperature while stirring with the three-one motor, stirred with the three-one motor for 1 hour, and then added with calcium carbonate and stirred to disperse the calcium carbonate sufficiently and uniformly to prepare a jelly-like capsule preparation solution. The calcium carbonate was prepared in advance as the 11 to 22 mass% of water dispersion, and homogenized (9,000 rpm, 15 minutes) using the homogenizer (manufactured by IKA; generator used: S25N-25F) before being added.

1-2. Method of forming film

Preparation of a cast film by the cooling gel method was performed as follows. A metallic applicator is placed on a glass surface or PET film kept at room temperature, and the preparation solution of 50 to 60°C is poured onto the glass surface or PET film and the applicator is moved at a constant speed to prepare a uniform film of 100 $\mu$m. Thereafter, the film was dried at room temperature to 30°C for about 10 hours.

Preparation of a cast film by the heating gel method was performed as follows. The metallic applicator is placed on the glass surface or PET film kept at 60°C, and the preparation solution of room temperature is poured onto the glass surface or PET film and the applicator is moved at a constant speed to prepare a uniform film of 100 $\mu$m. Thereafter, the film was dried at 60°C for 1 hour and then dried at room temperature for about 10 hours. In order to obtain the uniform film thickness of 100 $\mu$m, applicators having a gap of 0.4 mm to 1.5 mm were used as appropriate. The film thickness of the prepared cast film was within 100 $\mu$m $\pm$ 10 $\mu$m.

1-3. Measurement of transmittance

Evaluations of light-shielding ability were performed by evaluating transmittance using a UV-visible spectrophotometer (UV-1850, SHIMADZU CORPORATION). Transmittance was calculated by dividing a value of intensity of transmitted light when a sample film was measured by a value of intensity of transmitted light of a sample blank where the cast film is not placed. The cast film after drying was cut into strips of 10 mm $\times$ 20 mm, and as a sample film, placed vertically so as to shield a light path of the UV-visible spectrophotometer, and the light intensity of the transmitted light was measured to calculate the transmittance at measurement wavelengths of 650 nm and 420 nm.

1-4. Evaluation of strength

The cast film prepared by the above method was cut into a 5 mm x 75 mm dumbbell shape (as specified in JIS K-7161-2-1BA) to make a dumbbell-shaped test piece. The dumbbell-shaped test piece was placed in a glass desiccator containing a saturated salt solution, and the desiccator was kept in a thermostat bath at 25°C for at least one week to condition humidity. A saturated salt solution of potassium acetate was used as a low humidity condition (25°C, 22%

RH).

A tensile test was performed using the dumbbell-shaped test piece conditioned in the desiccator as described above at a tensile speed of 50 mm/min and a distance between chucks of 59 mm. The machine used was the EZ-LX, universal testing machine, manufactured by SHIMADZU CORPORATION. Elongation of the test piece and test force applied during the test were evaluated by the tensile test. A nominal stress $\sigma$ was obtained by dividing the test force by the initial cross-sectional area, and a nominal strain was obtained by dividing the elongation of the test piece by the initial length, respectively. Toughness (MJ/m$^3$) of the film was obtained from an area under a stress-strain curve obtained.

The toughness was measured eight times for each test piece, and a mean value and standard deviation (1SD) were obtained.

2. Results

Tables 2-1 and 2-2 show compositions of the standard, examples, and comparative examples, as well as transmittance at 420 nm and 650 nm, transmittance when the film thickness is converted to 100 $\mu$m, toughness, and standard deviation of the toughness.

Table 2-1

| Formulation | Type | Standard | Comparative example 1 | Example 1 | Example 2 | Example 3 | Example 4 | Example 5 | Comparative example 2 | Comparative example 3 | Example 6 | Example 7 | Example 8 |
|---|---|---|---|---|---|---|---|---|---|---|---|---|---|
| Hypromellose (percentage when total amount of hypromellose is set to 100 (parts by mass)) | 4mPa·s | 20 | 20 | 20 | 20 | 20 | 20 | 20 | 20 | 20 | | | 20 |
| | 4.5mPa·s | 20 | 20 | 20 | 20 | 20 | 20 | 20 | 20 | 20 | 20 | 20 | 20 |
| | 6mPa·s | 60 | 60 | 60 | 60 | 60 | 60 | 60 | 60 | 60 | 80 | 80 | 60 |
| HPMC | Viscosity value mPa·s | 530 | 530 | 530 | 530 | 530 | 530 | 530 | 530 | 530 | 570 | 570 | 530 |
| HPMC (mass%) | | Remainder | Remainder | Remainder | Remainder | Remainder | Remainder | Remainder | Remainder | Remainder | Remainder | Remainder | Remainder |
| κ-carrageenan (mass%) | | 1.5 | 1.5 | 1.5 | 1.5 | 1.5 | 1.5 | 1.5 | 1.5 | 1.5 | 1.5 | 1.5 | 0 |
| Potassium chloride (mass%) | | 0.44 | 0.44 | 0.44 | 0.44 | 0.44 | 0.44 | 0.44 | 0.44 | 0.44 | 0.44 | 0.44 | 0 |

(continued)

| Formulation | Type | | | | Standard | Comparative example 1 | Example 1 | Example 2 | Example 3 | Example 4 | Example 5 | Comparative example 2 | Comparative example 3 | Example 6 | Example 7 | Example 8 |
|---|---|---|---|---|---|---|---|---|---|---|---|---|---|---|---|---|
| | Manufacturer name | Product name | Dv50 (µm) | Heavy or precipitated | | | | | | | | | | | | |
| calcium carbonate (mass%) | BIHOKU FUNKA KOGYO | Precipitated calcium | 2.16 | Heavy | | | | | | | | | | | | |
| | CALFINE | Aragen | 5.05 | Heavy | | 7 | | | | | | | | | | |
| | YABASHI INDUSTRIES | Calpin F | 2.63 | Precipitated | | | 7 | | | | | | | | | 7 |
| | MARUO CALCIUM | CALTEX 5 | 1.82 | Heavy | | | | 7 | | | | | | | | |
| | CALFINE | ACE-35 | 2.04 | Heavy | | | | | | | | | | | 7 | |
| | BIHOKU FUNKA KOGYO | CALMIGEN A | 2.12 | Heavy | | | | | 7 | | | | | 7 | | |
| | MARUO CALCIUM | NANOX#25A | 3.02 | Heavy | | | | | | 7 | | | | | | |
| | MARUO CALCIUM | NANOX#30 | 2.21 | Heavy | | | | | | | 7 | | | | | |
| | Shiraishi Kogyo Kaisha | Calessen I | 0.39 | Precipitated | | | | | | | | 7 | | | | |
| | MARUO CALCIUM | CAL·ACE-ST | 0.51 | Precipitated | | | | | | | | | 7 | | | |
| | SANKYO SEIFUN | CALCY-F | 2.44 | Heavy | | | | | | | | | | | | |
| Titanium oxide | | | | | 2 | | | | | | | | | | | |
| Transmittance at 420 nm (%) | | | | | 1.72 | 19.51 | 4.70 | 3.06 | 3.78 | 4.89 | 4.19 | 12.47 | 12.71 | 3.35 | 2.96 | 3.74 |
| Transmittance at 650 nm (%) | | | | | 1.44 | 27.44 | 12.19 | 9.52 | 9.03 | 10.35 | 10.38 | 39.43 | 35.96 | 7.77 | 6.87 | 11.98 |

16

(continued)

| Formulation | Type | Standard | Comparative example 1 | Example 1 | Example 2 | Example 3 | Example 4 | Example 5 | Comparative example 2 | Comparative example 3 | Example 6 | Example 7 | Example 8 |
|---|---|---|---|---|---|---|---|---|---|---|---|---|---|
| | Transmittance at 420 nm (film thickness is converted to 100 micrometers) (%) | 1.15 | 17.58 | 3.60 | 2.95 | 4.29 | 5.18 | 4.59 | 14.03 | 14.28 | 4.43 | 3.96 | 3.74 |
| | Transmittance at 650 nm (film thickness is converted to 100 micrometers) (%) | 0.95 | 25.27 | 10.15 | 9.30 | 9.91 | 10.82 | 11.09 | 41.56 | 38.10 | 9.59 | 8.57 | 11.98 |
| | Toughness (MJ/m3) | 3.37 | 2.34 | 2.65 | 2.99 | 3.23 | 3.78 | 3.51 | 2.66 | 1.54 | 4.13 | 4.10 | 2.58 |
| | Standard deviation of toughness | 1.07 | 0.30 | 0.86 | 0.85 | 0.54 | 0.19 | 0.40 | 0.31 | 0.23 | 0.62 | 0.84 | 0.63 |
| | Film thickness ($\mu$m) | 91 | 94 | 92 | 99 | 104 | 102 | 103 | 106 | 106 | 109 | 109 | 100 |

EP 4 643 880 A1

Table 2-2

| Formulation | Type | Standard | Example 9 | Example 10 | Example 11 | Example 12 | Example 13 | Example 14 | Comparative example 4 | Example 15 | Comparative example 5 | Comparative example 6 | Comparative example 7 |
|---|---|---|---|---|---|---|---|---|---|---|---|---|---|
| Hypromellose (percentage when total amount of hypromellose is set to 100 (parts by mass)) | 4mPa·s | 20 | 20 | 20 | | | | | | | | | |
| | 4.5mPa·s | 20 | 20 | 20 | 100 | 100 | | | | | | | |
| | 6mPa·s | 60 | 60 | 60 | | | 100 | 100 | 100 | 80 | 80 | 80 | 80 |
| | 15mPa·s | | | | | | | | | 20 | 20 | 20 | 20 |
| HPMC | Viscosity value mPa·s | 530 | 530 | 530 | 450 | 450 | 600 | 600 | 600 | 780 | 780 | 780 | 780 |
| HPMC (mass%) | | Remainder | Remainder | Remainder | Remainder | Remainder | Remainder | Remainder | Remainder | Remainder | Remainder | Remainder | Remainder |
| κ-carrageenan (mass%) | | 1.5 | 0 | 1.5 | 1.5 | 1.5 | 1.5 | 1.5 | 1.5 | 1.5 | 1.5 | 1.5 | 1.5 |
| Potassium chloride (mass%) | | 0.44 | 0 | 0.44 | 0.44 | 0.44 | 0.44 | 0.44 | 0.44 | 0.44 | 0.44 | 0.44 | 0.44 |

(continued)

| Formulation | Type | | | | Standard | Example 9 | Example 10 | Example 11 | Example 12 | Example 13 | Example 14 | Comparative example 4 | Example 15 | Comparative example 5 | Comparative example 6 | Comparative example 7 |
|---|---|---|---|---|---|---|---|---|---|---|---|---|---|---|---|---|
| Calcium carbonate (mass%) | Manufacturer name | Product name | Dv50 ($\mu$m) | Heavy or precipitated | | | | | | | | | | | | |
| | BIHOKU FUNKA KOGYO | Precipitated calcium | 2.16 | Heavy | | | | | | | | | | | | |
| | CALFINE | Aragen | 5.05 | Heavy | | | | | | | | | | | | |
| | YABASHI INDUSTRIES | Calpin F | 2.63 | Precipitated | | | | | | | | | | | | |
| | MARUO CALCIUM | CALTEX 5 | 1.82 | Heavy | | | | | | | | | | | | |
| | CALFINE | ACE-35 | 2.04 | Heavy | | 7 | | | | 15 | 20 | 25 | | | | |
| | BIHOKU FUNKA KOGYO | CALMIGEN A | 2.12 | Heavy | | | | 6.3 | 13.6 | | | | | 20 | 25 | 30 | 40 |
| | MARUO CALCIUM | NANOX#25A | 3.02 | Heavy | | | | | | | | | | | | |
| | MARUO CALCIUM | NANOX#30 | 2.21 | Heavy | | | | | | | | | | | | |
| | Shiraishi Kogyo Kaisha | Calessen I | 0.39 | Precipitated | | | | | | | | | | | | |
| | MARUO CALCIUM | CAL·ACE-ST | 0.51 | Precipitated | | | | | | | | | | | | |
| | SANKYO SEIFUN | CALCY-F | 2.44 | Heavy | | | 7 | | | | | | | | | |
| Titanium oxide | | | | | 2 | | | | | | | | | | | |
| Transmittance at 420 nm (%) | | | | | 1.72 | 3.50 | 4.90 | 4.23 | 1.17 | 0.95 | 0.76 | 0.69 | 0.87 | 0.74 | 0.72 | 0.63 |
| Transmittance at 650 nm (%) | | | | | 1.44 | 8.30 | 8.14 | 8.89 | 2.12 | 1.53 | 1.03 | 0.85 | 1.30 | 0.97 | 0.92 | 0.72 |

(continued)

| Formulation | Type | Standard | Example 9 | Example 10 | Example 11 | Example 12 | Example 13 | Example 14 | Comparative example 4 | Example 15 | Comparative example 5 | Comparative example 6 | Comparative example 7 |
|---|---|---|---|---|---|---|---|---|---|---|---|---|---|
| | Transmittance at 420 nm (film thickness is converted to 100 micrometers) (%) | 1.15 | 3.16 | 5.19 | 4.78 | 1.33 | 1.18 | 1.09 | 0.95 | 0.75 | 0.74 | 0.57 | 0.43 |
| | Transmittance at 650 nm (film thickness is converted to 100 micrometers) (%) | 0.95 | 7.69 | 8.55 | 9.75 | 2.38 | 1.87 | 1.44 | 1.16 | 1.13 | 0.97 | 0.74 | 0.50 |
| | Toughness (MJ/m3) | 3.37 | 2.61 | 4.27 | 3.37 | 2.44 | 3.57 | 2.12 | 1.38 | 2.27 | 1.83 | 1.10 | 0.43 |
| | Standard deviation of toughness | 1.07 | 0.89 | 0.92 | 2.21 | 0.36 | 0.08 | 1.00 | 0.27 | 0.56 | 0.34 | 0.20 | 0.13 |
| | Film thickness ($\mu$m) | 91 | 97 | 102 | 104 | 103 | 105 | 108 | 107 | 97 | 100 | 96 | 93 |

The transmittance at the measurement light wavelength of 650 nm exceeded 20% in comparative examples 1 to 3, but was lower than 20% in examples 1 to 15. The transmittance at the measurement light wavelength of 420 nm exceeded 10% in comparative examples 1 to 3, but was lower than 10% in examples 1 to 15. In comparative examples 4 to 7, the transmittance was similar to that of the shell of examples, but the toughness was less than 2.0 MJ/m$^3$, and the strength of the film was inferior to that of the shell of examples. Therefore, it was considered preferable that the amount of calcium carbonate added is less than 25 mass%.

FIG. 1 shows a relationship between the transmittance and the Dv50 when the film thickness is converted to 100 μm graphically. FIG. 1A is a graph when the measurement light wavelength is 650 nm. The transmittance is 20% or less when the Dv50 is in a range of 0.9 to 3.8 μm. FIG. 1B is a graph when the measurement light wavelength is 420 nm. The transmittance is 10% or less when the Dv50 is in a range of 0.7 to 3.4 μm.

**Claims**

1. A hard capsule comprising a shell containing a cellulose compound and a light-shielding agent, wherein

   the light-shielding agent contains calcium carbonate having a median diameter by volume of 0.7 μm to 3.8 μm, and
   the content of the light-shielding agent in the shell is 3 to 20 mass% when the total of shell components of the hard capsule excluding moisture is set to 100 mass%.

2. The hard capsule according to claim 1, wherein the cellulose compound is hydroxypropyl methylcellulose.

3. The hard capsule according to claim 2, wherein the shell further contains (1) a gelling agent, or (2) the gelling agent and a gelling aid.

4. A hard capsule preparation solution containing a cellulose compound, a light-shielding agent and an aqueous solvent, wherein

   the light-shielding agent contains calcium carbonate having a median diameter by volume of 0.7 μm to 3.8 μm, and
   the content of the light-shielding agent in the total of components of a shell excluding the aqueous solvent is 3 to 20 mass% when the total of components of the preparation solution excluding the aqueous solvent is set to 100 mass%.

5. The hard capsule preparation solution according to claim 4, wherein the cellulose compound is hydroxypropyl methylcellulose.

6. The hard capsule preparation solution according to claim 5, further containing (1) a gelling agent, or (2) the gelling agent and a gelling aid.

7. A preparation method for a hard capsule including the following step:
   a step of preparing the hard capsule by a cooling gel method using the hard capsule preparation solution according to claim 4.

[FIG. 1]

(A)

(B)

## INTERNATIONAL SEARCH REPORT

| International application No. |
| --- |
| **PCT/JP2023/046599** |

| A. | CLASSIFICATION OF SUBJECT MATTER |
| --- | --- |

*A61K 47/02*(2006.01)i; *A23L 5/00*(2016.01)i; *A61K 8/11*(2006.01)i; *A61K 8/19*(2006.01)i; *A61K 8/20*(2006.01)i; *A61K 8/73*(2006.01)i; *A61K 9/48*(2006.01)i; *A61K 9/60*(2006.01)i; *A61K 9/62*(2006.01)i; *A61K 47/36*(2006.01)i; *A61K 47/38*(2006.01)i
FI: A61K47/02; A61K9/60; A61K47/38; A61K47/36; A61K8/11; A61K8/20; A61K8/19; A61K8/73; A61K9/62; A23L5/00 C; A61K9/48

According to International Patent Classification (IPC) or to both national classification and IPC

| B. | FIELDS SEARCHED |
| --- | --- |

Minimum documentation searched (classification system followed by classification symbols)

A61K47/00-47/69, A61K9/00-9/72

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | JP 2021-523917 A (CAPSUGEL BELGIUM NV) 09 September 2021 (2021-09-09) claims, paragraph [0029], examples, tables 1, 5 | 1-6 |
| Y | claims, paragraph [0029], examples, tables 1, 5 | 7 |
| Y | JP 2022-123274 A (QUALICAPS CO., LTD.) 24 August 2022 (2022-08-24) claims, paragraph [0010] | 7 |
| Y | JP 2022-549359 A (HERCULES LLC) 24 November 2022 (2022-11-24) claims, paragraph [0039], examples 5, 10 | 1-7 |
| Y | JP 7-124462 A (WARNER LAMBERT CO.) 16 May 1995 (1995-05-16) claims, paragraph [0005], example 3 | 1-7 |
| Y | WO 2021/145296 A1 (CAPSUGEL JAPAN INC.) 22 July 2021 (2021-07-22) paragraphs [0006], [0007] | 1-7 |

| ☐ Further documents are listed in the continuation of Box C. | ✓ See patent family annex. |
| --- | --- |

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **14 March 2024** | **26 March 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

# EP 4 643 880 A1

## INTERNATIONAL SEARCH REPORT
### Information on patent family members

International application No.

**PCT/JP2023/046599**

| Patent document cited in search report | | | Publication date (day/month/year) | Patent family member(s) | | | Publication date (day/month/year) |
|---|---|---|---|---|---|---|---|
| JP | 2021-523917 | A | 09 September 2021 | US | 2021/0052505 | A1 | |
| | | | | claims, paragraph [0032], examples, tables 1, 5 | | | |
| | | | | WO | 2019/219693 | A1 | |
| | | | | EP | 3758685 | A1 | |
| | | | | CN | 112118835 | A | |
| JP | 2022-123274 | A | 24 August 2022 | (Family: none) | | | |
| JP | 2022-549359 | A | 24 November 2022 | US | 2022/0347103 | A1 | |
| | | | | claims, paragraph [0046], examples 5, 10 | | | |
| | | | | WO | 2021/062158 | A1 | |
| | | | | EP | 4033912 | A1 | |
| | | | | KR | 10-2022-0070473 | A | |
| | | | | CN | 114760846 | A | |
| JP | 7-124462 | A | 16 May 1995 | (Family: none) | | | |
| WO | 2021/145296 | A1 | 22 July 2021 | (Family: none) | | | |

Form PCT/ISA/210 (patent family annex) (July 2022)

24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008156027 A
- WO 2019219693 A
- WO 2021069590 A
- JP 2007144014 A
- JP 2000226097 A
- US 3508678 A
- US 3823843 A
- US 4040536 A
- US 4822618 A

- US 5769267 A
- JP 2005187412 A
- JP 2009504630 A
- US 4069819 A
- US 4210140 A
- US 7669596 B
- US 20100300440
- WO 2006070578 A

**Non-patent literature cited in the description**

- Aqueous Polymeric Coating For Pharmaceutical Dosage Forms. CRC Press, 2017